(19) Europäisches Patentamt
European Patent Office
Office européen des brevets

(11) Publication number : **0 246 506 B1**

# (12) EUROPEAN PATENT SPECIFICATION

(45) Date of publication of patent specification : **02.01.92 Bulletin 92/01**

(51) Int. Cl.$^5$ : **C10M 105/06,** C07C 15/24, // (C10M105/06, 105:00), (C10N40/04, 40:16)

(21) Application number : **87106655.1**

(22) Date of filing : **07.05.87**

## (54) Fluid composition, process for preparation thereof and its use as an electric insulating oil.

(30) Priority : **22.05.86 JP 116150/86**

(43) Date of publication of application : **25.11.87 Bulletin 87/48**

(45) Publication of the grant of the patent : **02.01.92 Bulletin 92/01**

(84) Designated Contracting States : **BE CH DE FR GB IT LI NL SE**

(56) References cited :
**EP-A- 0 164 038**
**EP-A- 0 224 259**
**DE-A- 3 524 188**
**FR-A- 2 529 227**
**GB-A- 2 091 289**
**GB-A- 2 168 378**
**US-A- 3 069 478**

(73) Proprietor : **IDEMITSU KOSAN COMPANY LIMITED**
**1-1, Marunouchi 3-chome Chiyoda-ku**
**Tokyo (JP)**

(72) Inventor : **Minokami, Tomiyasu**
**No. 1660, Kamiizumi Sodegaura-machi**
**Kimitsu-gun Chiba-ken (JP)**
Inventor : **Shimizu, Nobuaki**
**No. 1205-139 Kamiizumi Sodegaura-machi**
**Kimitsu-gun Chiba-ken (JP)**
Inventor : **Tsubouchi, Toshiyuki**
**No. 1660, Kamiizumi Sodegaura-machi**
**Kimitsu-gun Chiba-ken (JP)**

(74) Representative : **Türk, Gille, Hrabal**
**Brucknerstrasse 20**
**W-4000 Düsseldorf 13 (DE)**

**EP 0 246 506 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

The present invention relates to a 1-phenyl-1-naphthylethane composition, a process for preparation of said composition and its use. More particularly, it is concerned with a 1-phenyl-1-naphthylethane composition having excellent low temperature fluidity in which the ratio of α-form to β-form is controlled to a specified value by choosing the molar ratio of starting materials to be fed, the type of a catalyst and the reaction temperature, a process for efficiently preparing said composition, and its use as an electirc insulating oil or as a heat medium.

Heretofore 1-phenyl-1-naphthylethane has been produced by reacting naphthalene and styrene in the presence of a Friedel-Craft catalyst such as sulfuricacid (Ber. 65B, p. 1686 (1932) and JP-A- 32924/1977).

This method using the Friedel-Craft catalyst, however, has various disadvantages; for example, (1) a reactor is readily corroded, (2) after the reaction, the catalyst and the desired product are difficult to separate, (3) post-treatment (e.g., neutralization and washing) of the reaction product is needed, and (5) there is a danger of enviromental pollution due to waste water.

Furthermore, 1-phenyl-1-naphthylethane produced by the above method contains 1-phenyl-1-(α-naphthyl)ethane in a ratio of 70% or more and thus suffers from a serious disadvantage that it solidifies at room temperature because of its poor fluidity at low temperature. The same is valid for EP-A-0164083 wherein a mixture of 1-(1-naphthyl)-1-phenyl ethane and 1-(2-naphthyl)-1-phenyl ethane is described wherein the α-form/β-form ratio is 80/20.

A method of reacting naphthalene and α-methylstyrene in the presence of activated clay or acidic clay (JP-A-17526/1986) has a disadvantage in that dimerization of α-methylstyrene occurs and thus the yield of the desired product is low.

A method of reacting tetralin and styrene in the presence of clays (JP-B- 33692/1980) has a disadvantage in that the styrene content is not more than 6% by weight and the proportion of α-form formed is less than 10%.

A method of reacting an alkyl benzene or an alkyl naphthalene and styrene in the presence of acidic clay (US-A- 3 069 478) is intended to obtain a product having good fluidity by choosing the above starting materials. However it has a disadvantage in that methylnaphthalene as one of the starting materials contains a nitrogen compound which is a catalyst poison and that this process cannot be carried out by using unsubstituted aromatic hydrocarbons as starting material.

### SUMMARY OF THE INVENTION

The object of the present invention is to overcome the above problems of the prior art, and to provide a fluid composition containing 1-phenyl-1-(α-naphthyl)ethane (α-form) and 1-phenyl-1-(β-naphthyl)ethane (β-form) in the specified ratio and also a process for efficiently preparing the above fluid composition.

It has been found that the above object can be attained by controlling the ratio of naphthalene and styrene to be fed as the starting materials and the reaction temperature.

The present invention relates to a fluid composition comprising 30 to 65% by weight of 1-phenyl-1-(α-naphthyl)-ethane and 70 to 35% by weight of 1-phenyl-1-(β-naphthyl)-ethane provided that the sum of the amounts of 1-phenyl-1-(α-naphthyl)ethane and 1-phenyl-1-(β-naphthyl)ethane is 100 %.

The present invention further relates to a process for preparing a fluid composition as defined above which comprises feeding naphthalene and styrene in a molar ratio of naphthalene to styrene of 1:1 to 10:1 and reacting them in the presence of a clay at a temperature of from 50 to 180°C.

Finally, the present invention relates to the use of the fluid composition as defined above as an electric insulating oil or as a heat medium.

### BRIEF DESCRIPTION OF THE DRAWINGS

Fig. 1 is a graph showing a relation between traction coefficient of each of the fluids obtained in Reference Example 5 and Comparative Example 5 and temperature; and Fig. 2 is a graph showing a relation between traction coefficient of each of the fluids obtained in Reference Example 6 and Comparative Example 6 and temperature.

### DETAILED DESCRIPTION OF THE INVENTION

Naphthalene which is one of the starting materials to be used in the present invention may be naphthalene derived from coal or naphthalene derived from petroleum and can be produced by any desired method. In addition, naphthalene on the market can be used.

Styrene which is to be used as the other starting material in the present invention can be produced by any desired method. In addition, styrene on the market can be used.

The molar ratio of naphthalene to styrene to be feed in the present invention is 1:1 to 10:1. If the molar ratio of naphthalene to styrene is less than 1:1, diaralkylation or polymerization of styrene monomers is accelerated and thus the yield of the desired product, 1-phenyl-1-naphthylethane, is decreased. On the other hand, if the molar ratio is in excess of 10:1, the proportion of the desired product in the reaction product is increased, but the amount of the reaction product is small, which is undesirable from a practical standpoint. If the molar ratio of naphthalene to styrene is increased, the 1-phenyl-1-($\alpha$-naphthyl)ethane content of the desired product tends to be increased.

In accordance with the present invention, the above naphthalene and styrene are reacted at the specified temperature in the presence of clays as the catalyst. As these clays, various compounds can be used. In particular, bentonite, montmorillonite, acid clay, activated clay, kaolin and so forth are preferred. The most preferred is activated clay. These clays can be used in various forms such as a powdered form, a granular form, a slice form, a pellet form and a spherical form.

These clays are used as such or alternatively they are treated with mineral acids such as sulfuric acid, hydrochloric acid, phosphoric acid, hydrobromic acid and hydrofluoric acid prior to the use thereof in order to more increase catalytic activity. The clays are usually dried or burned at a temperature of from 100 to 600°C, preferably from 200 to 400°C and then used.

The amount of the clays used varies with the amount of the starting materials used, the reaction temperature, the pressure and so forth. Usually the amount of the clays used is not less than 0.2% by weight, preferably from 1 to 10% by weight based on the weight of all the starting materials. If the amount of the clays used is less than 0.2% by weight, the reaction rate is decreased and thus a long time is needed for the reaction, and furthermore the yield of the desired product is undesirably decreased. On the other hand, even if the amount of the clays used is increased, the reaction is not hindered. However, from an economic standpoint, the clays is used in an amount of not more than 10% by weight.

In the present invention, the reaction temperature should be controlled to from 50 to 180°C and preferably from 80 to 150°C. If the reaction temperature is less than 50°C, the reaction rate is decreased. On the other hand, if the reaction temperature is more than 180°C, the 1-phenyl-1-($\alpha$-naphthyl)ethane content of the 1-phenyl-1-naphthylethane product is outside the range of from 30 to 65 mol% (i.e., less than 30 mol%). Thus the low temperature fluidity of the product is decreased and further the product is undesirably decomposed.

As the reaction temperature is decreased, the 1-phenyl-1-($\alpha$-naphthyl)ethane content of the product tends to be increased.

In reacting naphthalene and styrene in accordance with the process of the present invention, it is preferred for the naphthalene and styrene to be intimately mixed in the presence of the clay catalyst. For this reason, the concentration of styrene in the reaction system is preferably not more than 10% by weight and most preferably not more than 5% by weight. If the styrene concentration is too high, side reactions such as polymerization of styrene occur, resulting in a reduction in the yield of the desired product.

In the present invention, the reaction procedure is not critical as long as the above requirements are met. For example, any of batchwise, semibatchwise or flowwise method can be used. In accordance with a preferred embodiment of the present invention, naphthalene and the clays are introduced in a reactor and then styrene is added to the reaction mixture in small portions while stirring. After the completion of addition of styrene, it is preferred for the reaction mixture to be subjected to post-stirring. The reaction time including the times for addition of styrene and for stirring after the addition is from about 30 minutes to 24 hours. In post-stirring, it is preferred that the temperature is maintained within the above reaction temperature range and the stirring is carried out while maintaining the above predetermined temperature. The reaction pressure is not critical. Usually the reaction is carried out at self pressure at the reaction temperature or at atmospheric pressure. The reaction can be carried out in an atmosphere of inert gas (e.g., nitrogen gas, hydrogen gas and argon gas). In addition, the reaction can be carried out in the presence of a suitable inert solvent. Particularly in a case where the reaction is carried out at a relatively low temperature (below melting point of naphthalene), it is preferred that naphthalene be dissolved in the inert solvent and reacted. As the inert solvent, aliphatic saturated hydrocarbons, alicyclic saturated hydrocarbons or their halogenated compounds are preferably used. Representative examples are pentanes, hexanes, octanes, cyclohexanes, methylcyclohexanes, ethylcyclohexanes, and decalins. In addition, f.e. carbon tetrachloride, 1,2-dichloroethane, and 1,1,2-trichloroethane can be used.

After the completion of the reaction, the clays are removed from the reaction mixture by suitable techniques such as filtration and centrifugal separation. The reaction mixture thus freed of the clays is, as such or after washing with water, subjected to usual distillation, preferably vacuum distillation to fractionate it, whereupon the desired 1-phenyl-1-naphthylethane fluid composition containing the $\alpha$-form and the $\beta$-form in the specified

ratio can be obtained.

The fluid composition thus obtained is a mixture containing 1-phenyl-1-(α-naphthyl)ethane having the formula:

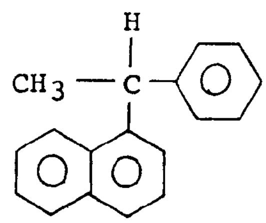

(α-form) and 1-phenyl-1-(β-naphthyl)ethane having the formula:

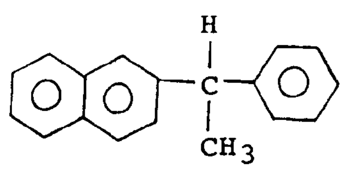

(β-form) as the main components and a fraction containing them. In this composition, the α-form content is 30 to 65 mol%, preferably 40 to 55 mol% based on the total amount of the α-form and the β-form. If the α-form content is not within the above range, the low temperature fluidity of the composition is decreased. The α- and β-forms each have the following high melting points (i.e., solid at ordinary temperature):

Melting point of the α-form: 65-66°C
Melting point of the β-form: 37-38°C
(according to J. Chem. Soc., P. 1935 (1948)).

If, however, the α- and β-forms are mixed in the above specified ratio, the low temperature fluidity is markedly increased owing to the drop of melting point. Test results of the relation between the contents of the α- and β-forms and the fluidity of the mixture are shown in Table 1 below.

Table 1

| | | | | |
|---|---|---|---|---|
| α-Form (mol%) | 72 | 58 | 44 | 20 |
| β-Form (mol%) | 28 | 42 | 56 | 80 |
| Pour Point of Mixture (°C) | +15 | -11 | -20 | +10 |

The 1-phenyl-1-naphthylethane composition obtained by the method of the present invention contains, as described above, the α-form in a proportion of from 30 to 65 mol%. Thus the composition is low in flow point (flow point of not more than 0°C) and is excellent in low temperature fluidity.

In accordance with the process of the present invention, the 1-phenyl-1-naphthylethane composition can be prepared in a simplified manner and further in a high yield as compared with the conventional methods. Furthermore, since the reaction mixture is neutral in the process of the present invention, the reaction unit is not corroded and the step of neutralization and washing of the reaction product is not needed, and thus there is no danger of enviromental pollution due to waste water. Furthermore the process of the present invention has an advantage in that the catalyst can be easily separated from the reaction mixture.

The fluid composition of the present invention is, as described above, excellent in low temperature fluidity and has good hydrogen gas absorption properties, and thus it is useful as an electric insulating oil. In this case, the fluid composition can be used alone or in combination with known electric insulating oils such as mineral oils, polybutenes, alkylbenzenes and aromatic hydrocarbons having two or three aromatic rings. To the fluid composition of the present invention, lubricating oil additives such as an antioxidant, a detergent dispersant, a rust inhibitor, an emulsifying agent, an antifoaming agent and an extreme pressure agent can be added. The

fluid composition of the present invention is excellent in thermal stability, and thus it can be used alone or in combination with other mineral oils or synthetic oils as a thermal medium oil. In this case, commonly used lubricating oil additives can be added. In addition, the fluid composition of the present invention can be used as an intermediate material for preparation of fluid for traction drive, a plasticizer for rubber or various synthetic resins, a solvent for pressure-sensitive paper, and so forth.

Conditions under which the fluid composition of the present invention is hydrogenated to produce a fluid for traction drive will hereinafter be explained.

Hydrogenation conditions are controlled so that the reaction temperature is not more than 300°C and the reaction time is within the range of 10 minutes to 10 hours. The above reaction is usually at a hydrogen pressure of 0,1 to 25,0 MPa (1 to 250 kg/cm$^2$). It is particularly advantageous to control the hydrogen pressure within the range of 0,5 to 15,8 MPa (5 to 150 kg/cm$^2$) because if so the reaction proceeds smoothly and no special reaction equipment is needed.

The above reaction is carried out in the presence of a catalyst. As this catalyst, those commonly used as hydrogenation catalysts can be used. These hydrogenation catalysts include a Ni catalyst, a Ru catalyst, a Rh catalyst, a Pd catalyst and a Pt catalyst. In particular, a catalyst containing at least one metal component of the Ru, Rh and Pt catalysts is preferably used in the present invention.

Examples of the Ru catalyst include ruthenium chloride, ammonium chlororuthenate, ruthenium hydroxide, ruthenium oxide, ruthenium dioxide, ruthenium tetraoxide and potassium ruthenate. These compounds are used as such or alternatively in the form that is deposited on a carrier such as activated carbon. Examples of the Rh catalyst include rhodium chloride, sodium chlororhodinate, ammonium chlororhodinate, rhodium (III) hydroxide, rhodium (IV) hydroxide, and rhodium (III) oxide. These compounds can be used as such or alternatively in the form that is supported on a carrier. Examples of the Pt catalyst include a combination of platinum black, platinum chloride and tetrachloroplatinic acid, a combination of platinum black, platinum chloride and hexachloroplatinic acid, a combination of ammonium hexachloroplatinate, platinum oxide, platinum hydroxide, platinum dioxide, platinum disulfide and platinum sulfide, and a combination of platinum disulfide and platinum sulfide. These compounds may be supported on a carrier (e,g., a platinum/carbon catalyst, a platinum/asbestos catalyst, a platinum/silica gel catalyst and a platinum/alumina catalyst).

Of these catalysts, the ruthenium/activated carbon catalyst is preferable.

The amount of the catalyst used is controlled within the range of from 0.01 to 10 parts by weight, preferably from 3 to 7 parts by weight per 100 parts by weight of 1-phenyl-1-naphthylethane ($\alpha$-form: 30 to 60% by weight). If the amount of the catalyst used is less than 0.01 part by weight, the reaction time is increased. On the other hand, even if the amount of the catalyst used is more than 10 parts by weight, no marked increase in the reaction efficiency is obtained.

Since 1-phenyl-1-naphthylethane ($\alpha$-form: 30 to 65% by weight) is obtained in a liquid form, the above reaction can be carried out without the use of a solvent. However, in carrying the above reaction, 1-phenyl-1-naphthylethane ($\alpha$-form: 30 to 65% by weight) can be dissolved or dispersed in the solvent. In this case, a solvent capable of dissolving the 1-phenyl-1-naphthylethane ($\alpha$-form: 30 to 65% by weight) can be used. Solvents which can be used include saturated hydrocarbon solvents (e.g., hexane, cyclohexane and methylcyclohexane), aromatic hydrocarbon solvents (e.g., benzene, toluene and xylene), ester solvents (e.g., methyl acetate and ethyl acetate), alcohol solvents (e.g., methyl alcohol and ethyl alcohol), and ketone solvents (e.g., acetone and methyl ethyl ketone).

The fluid obtained by hydrogenation of the fluid composition of the present invention can be used as a fluid for traction drive.

In a case where the fluid composition of the present invention is used as a fluid for traction drive, other compounds for traction drive and, if necessary, various additives can be compounded thereto.

Other compounds for traction drive are not critical. Representative examples of the compounds are 1,3-dicyclohenyl-3-methylbutane, 1,2-di(dimethylcyclohexyl)propane, hydrogenated product of liner dimer of methylstyrene, cyclohexylmethyl decalin, 1-(methyldecalyl)-1-cyclohexylethane, cyclohexylmethyl perhydrofluorene, cyclohexylmethyl perhydroacenaphthene, bisdecalin, 1,3,5-tricyclohexyl-5-methylhexane, tercyclohexyl, and 1,1,3-trimethyl-3-cyclohexylhydrindane,.

The present invention is described in greater detail with reference to the following examples.

PREPARATION EXAMPLE 1

Weak Acid Type Catalyst

100 g of activated clay (Galleonearth NS produced by Mizusawa Kagaku Kogyo Co., Ltd., powdered form) was calcined at 110°C for 3 hours and then at 250°C for 8 hours to obtain the desired catalyst.

PREPARATION EXAMPLE 2

Strong Acid Type Catalyst

100 g of activated clay (Galleonite 308D produced by Mizusawa Kagaku Kogyo Co., Ltd., molded form) was calcined at 110°C for 3 hours and then at 250°C for 8 hours.

EXAMPLES 1 TO 5, AND COMPARATIVE EXAMPLE 1

512 g of naphthalene and 51.2 g (10% by weight of the naphthalene) of the catalyst prepared in Preparation Example 1 were placed in a 1-liter glass reactor equipped with a dropping funnel, a reflux condenser, stirrer and a thermometer. The inside of the reactor was maintained at a temperature higher than the melting point (80.3°C) of naphthalene. The contents was raised to the temperature shown in Table 2, while stirring. While maintaining the contents at the above elevated temperature, 104 g of styrene was dropped with stirring over 2 hours. After the completion of addition, the contents were further stirred for 30 minutes.

After the completion of the reaction, the catalyst was separated or removed from the reaction mixture. Then the reaction mixture was vacuum distilled to obtain a fraction having a boiling point of 127-143°C [26,7 Pa (0.2 mmHg)]. Almost all of the fraction (desired product) was 1-phenyl-1-naphthylethane. The yield and properties of the desired product are shown in Table 2.

Table 2

| | Example 1 | Example 2 | Example 3 | Example 4 | Example 5 | Comparative Example 1 |
|---|---|---|---|---|---|---|
| Charged Amount (g) | | | | | | |
| Naphthalene | 512 | 512 | 512 | 512 | 512 | 512 |
| Styrene | 104 | 104 | 104 | 104 | 104 | 104 |
| Catalyst | 51.2 | 51.2 | 51.2 | 51.2 | 51.2 | 51.2 |
| Molar Ratio of Charge *1 | 4/1 | 4/1 | 4/1 | 4/1 | 4/1 | 4/1 |
| Reaction Temperature (°C) | 84 | 102 | 123 | 148 | 173 | 198 |
| Time for Dropping of Styrene (hrs) | 2 | 2 | 2 | 2 | 2 | 2 |
| Desired Product | | | | | | |
| α-Form Content (%) | 57 | 56 | 53 | 51 | 40 | 8 |
| Yield *2 | 70 | 72 | 76 | 78 | 71 | 62 |
| Pour Point (°C) | -12 | -13 | -15 | -16 | -13 | +25 |

*1 Molar ratio of naphthalene/styrene

*2 Mol% of the formed 1-phenyl-1-naphthylethane based on the styrene starting material

EXAMPLES 6 AND 7, AND COMPARATIVE EXAMPLE 2

The procedure of Example 4 was repeated with the exception that the charged amounts of naphthalene, styrene and catalyst, and the time for dropping styrene were changed as shown in Table 3. The results are shown in Table 3.

Table 3

| | Example 6 | Example 7 | Comparative Example 2 |
|---|---|---|---|
| Charged Amount (g) | | | |
| Naphthalene | 640 | 384 | 128 |
| Styrene | 52 | 208 | 312 |
| Catalyst | 64.0 | 38.4 | 12.8 |
| Molar Ratio of Charge *1 | 10/1 | 1.5/1 | 1/3 |
| Reaction Temperature (°C) | 148 | 148 | 148 |
| Time for Dropping of Styrene (hrs) | 1 | 4 | 6 |
| Desired Product | | | |
| α-Form Content (%) | 58 | 52 | 22 |
| Yield *2 | 89 | 58 | 30 |
| Pour Point (°C) | -11 | -16 | +11 |

*1, *2 Same as in Table 2.

EXAMPLE 8

The procedure of Example 4 was repeated with the exception that 25.6 g of the catalyst prepared in Preparation Example 2 was used in place of the catalyst prepared in Preparation Example 1. The results are shown in Table 4.

COMPARATIVE EXAMPLE 3

192 g of naphthalene and 192 g of carbon tetrachloride were placed in a 1-liter glass reactor equipped with a dropping funnel, a stirrer and a thermometer and stirred for 30 minutes. Then 19.2 g of sulfuric acid having a concentration of 95% was introduced, and 104 g of styrene was dropped with stirring over 2 hours while maintaining the temperature at 20°C. After the completion of dropwise addition, the mixture was further stirred for 30 minutes at 20°C.

After the completion of the reaction, the sulfuric acid layer was separated from the reaction mixture, and the resulting mixture was neutralized with sodium hydroxide, further washed with water and subjected to dehydration treatment, and then vacuum distilled to obtain a fraction having a boiling point of 127-143°C [26,7 Pa (0.2 mmHg)]. This fraction (desired product) was substantially composed of 1-phenyl-1-naphtylethane. The yield and properties of the desired product are shown in Table 4.

Table 4

| | Example 8 | Comparative Example 3 |
|---|---|---|
| Charged Amount (g) | | |
| Naphthalene | 512 | 192 |
| Styrene | 104 | 104 |
| Catalyst | 25.6 | 19.2 |
| Molar Ratio of Charge *1 | 4/1 | 1.5/1 |
| Reaction Temperature (°C) | 148 | 20 |
| Time for Dropping of Styrene (hrs) | 2 | 2 |
| Desired Product | | |
| α-Form (%) | 50 | 72 |
| Yield *2 (%) | 74 | 51 |
| Pour Point (°C) | -16 | +15 |

*1, *2 Same as in Table 2.

REFERENCE EXAMPLE 1

512 g (4 mol) of naphthalene and 51.2 g of activated clay (Galleonearth NS produced by Mizusawa Kagaku Kogyo Co., Ltd.) which had been dried at 250°C for 6 hours were placed in a 1-liter four-necked flask equipped with a stirrer, a thermometer, a dropping funnel provided with a side tube and a Dimroth reflux condenser. Then 102 g (1 mol) of styrene was dropped with stirring at 152°C over 3 hours, and the resulting mixture was further stirred for 30 minutes to complete the reaction. The reaction mixture was immediately filtered to separate the catalyst and then vacuum distilled to obtain 180 g of a fraction having a boiling point of 147-152°C [14,7 Pa (0.11 mmHg)]. An analysis showed that the fraction was a mixture of 43% of 1-phenyl-1-(α-naphthyl)ethane and 57% of 1-phenyl-1-(β-naphthyl)ethane. In connection with properties of the fluid, the kinematic viscosity was $28{,}08 \cdot 10^{-6}$ $m^2/s$ (28.08 centistokes (cSt)) (40°C) and $3{,}317 \cdot 10^{-6}$ $m^2/s$ (3.317 cSt) (100°C) ; the specific gravity was 1.0717 (15/4°C); the pour point was -20°C; the refractive index was 1.5700 or more ($n_D^{20}$); and the flash point was 206°C (COC).

The above mixture was measured for properties as an electric insulating oil. The results are shown in Table 5.

A predetermined amount of the above mixture was added to a lubricating oil having a boiling point of 250-420°C as a mineral oil, and the resulting mixture was measured for properties as an electric insulating oil. The results are shown in Table 5.

COMPARATIVE EXAMPLE 4

A mineral oil having a boiling point of 250-420°C was measured for properties as an electric insulating oil. The results are shown in Table 5.

Table 5

| | | Reference Example 1 | | | Comparative Example 4 |
|---|---|---|---|---|---|
| 1-Phenyl-1-Naphthylethane/Mineral Oil | | 100/0 | 30/70 | 5/95 | 0/100 |
| Physical Properties | | | | | |
| Specific Gravity | 15/4°C | 1.0717 | 0.9300 | 0.8794 | 0.8693 |
| Flash Point | (COC) °C | 206 | 174 | 170 | 168 |
| Pour Point | °C | -20 | -25 | -30 | -30 |
| Kinematic Viscosity | (40°C) ~~cSt~~ $10^{-6}$ $m^2/s$ (cSt) | 28.08 | 11.472 | 8.616 | 8.242 |
| Hydrogen Gas Absorption* (mm) | | -298 | -97 | -20 | +14 |
| Oxidation Stability (120°C x 75 hrs) | | | | | |
| Total Acid Value (mg KOH/g) | | 0.07 | 0.11 | 0.14 | 0.15 |
| Amount of Sludge (wt%) | | 0 | 0.04 | 0.06 | 0.08 |
| Volume Resistance (Ω·cm x $10^4$) | | 40 | 40 | 30 | 30 |
| Dielectric Loss (%) | | 0.003 | 0.003 | 0.003 | 0.004 |
| Dielectric Breakdown Voltage (kv/25 mm) | | ≧70 | ≧70 | ≧70 | ≧70 |

*Measured according to the method of Insulating Oil Associate (applied voltage: 8 kv; temperature: 50°C; time: 120 minutes).

Dielectric characteristics were measured according to JIS-C2101.

It can be seen from Table 5 that the composition of the present invention is an electric insulating oil having excellent hydrogen gas absorption properties even when used alone or in combination with a mineral oil.

REFERENCE EXAMPLE 2

The 1-phenyl-1-naphthylethane mixture obtained in Reference Example 1 was tested for lubricating oil thermal stability at 170°C for 24 hours according to JIS-K2540. Neither change in hue between before and after heating nor formation of sludge was observed.

REFERENCE EXAMPLE 3

The 1-phenyl-1-naphthylethane mixture of Reference Example 1 was subjected to the Falex test and evaluated as a refrigerating machine oil. The results are shown in Table 6. The mixture was excellent in compatibility with a refrigerant.

Table 6

| | Reference Example 3 |
|---|---|
| Falex Test *1 | |
| Seizure Load (lbs) | 640 |
| Two Layer Separation Temperature (°C) *2 | less than -55 |
| Sealed Tube Test *3 | |
| Cu Appearance | no change |
| Fe Appearance | no change |
| Al Appearance | no change |
| Oil Appearance | no change |
| Sludge Formation | none |
| Amount of Formed Hydrochloric Acid (mg/4 ml) | 0.46 |
| Kinematic Viscosity | |
| 40°C . $10^{-6}$ $m^2/s$ (cSt) | 28.08 |
| 100°C . $10^{-6}$ $m^2/s$ (cSt) | 3.317 |

*1 According to ASTM D-3233.

*2 Value when the oil content of the mixture of refrigerant R-22 and oil was 15 wt%.

*3 4 ml of oil and 1.5-1.7 g of refrigerant R-12 were placed in a pressure glass tube, a rod of Cu, Fe or Al was introduced as a catalyst, and the mixture was allowed to stand at 170°C for 200 hours.

REFERENCE EXAMPLE 4

The 1-phenyl-1-naphthylethane mixture obtained in Reference Example 1 was subjected to the thermal decomposition test (sealed tube test) at 350°C x 5 days and 350°C x 10 days in the sealed condition. The results

are shown in Table 7.

Table 7

| | Fresh Oil | 5 Days | 10 Days |
|---|---|---|---|
| Kinematic Viscosity (cSt) 40°C | 28.08 | 27.81 | 27.57 |
| Viscosity Ratio(%) | ___ | -0.96 | -1.82 |
| Kinematic Viscosity (cSt) 100°C | 3.317 | 3.283 | 3.254 |
| Viscosity Ratio(%) | ___ | -1.03 | -1.90 |

1 cSt = $10^{-6}$ $m^2/s$

It can be seen from the above results that the composition of the present invention is a heat medium oil which is decrease in thermal decomposition and excellent in thermal stability. Furthermore the composition of the present invention has characteristics that it does not corrode metals, has a low vapour pressure, and has a high flash point.

REFERENCE EXAMPLE 5

150 g of 1-phenyl-1-naphthylethane (a mixture of 65 vol.% of 1-phenyl-1-(1-naphthyl)ethane and 35 vol.% of 1-phenyl-1-(2-naphthyl)ethane) and 12 g of a 5% ruthenium-carbon catalyst (produced by Nippon Engelhard Co., Ltd.) were placed in a 1-liter autoclave and hydrogenated at a hydrogenation pressure of 8,1 MPa (80 $kg/cm^2G$) and a reaction temperature of 150°C for 4 hours. The catalyst was separated by filtration to obtain 155 g of 1-cyclohexyl-1-decalylethane. A nuclear magnetic resonance (NMR) spectral analysis showed that the degreee of hydrogenation was more than 99% and the product was a mixture of 65 vol.% of 1-cyclohexyl-1-(1-decalyl)ethane and 35 vol.% of 1-cyclohexyl-1-(2-decalyl)ethane. The kinematic viscosity was $39.64 \cdot 10^{-6}$ $m^2/s$ (39.64 cSt) at 40°C and $4,629 \cdot 10^{-6}$ $m^2/s$ (4.629 cSt) 100°C; the specific gravity was 0.9458 (15/4°C); the refractive index was 1.5061 ($n_D^{20}$). The traction coefficient was measured within the range of 40-140°C. The results are shown in Fig. 1.

The traction coefficient was measured using a two roller machine. One of two rollers which were of the same size and in contact with each other (diameter: 52 mm; thickness: 6 mm; the roller to be driven was of the barrel-shape form that the curvature was 10 mm and the driving roller was of the flat type without crowing)was continuously rotated at a constant rate (1,500 rpm) and the other at a rate ranging between 1,500 rpm and 1,750 rpm. A load of 7 kg was applied with spring to a contact area between the two rollers, and a tangential force generated between the two rollers, i.e., traction force was measured to determine a traction coefficient. These rollers were made of Bearing Steel SUJ-2 and mirror finished, and the maximum Hertzian contact pressure was 112 $kgf/mm^2$.

In measuring a relation between traction coefficient and oil temperature, the oil temperature was changed from 40°C to 140°C by heating the oil tank with a heater and a relation between traction coefficient at a slip ratio of 5% and oil temperature was plotted.

COMPARATIVE EXAMPLE 5

The procedure of Reference Example 5 was repeated wherein a mixture of 95% of 1-phenyl-1-(β-naphthyl)ethane and 5% of 1-phenyl-1-(α-naphthyl)ethane was used, to thereby obtain 155 g of 1-cyclohexyl-1-decalylethane (a mixture of 95% of 1-cyclohexyl-1-(2-decalyl)ethane and 5% of 1-cyclohexyl-1-(1-decalyl)ethane) having a kinematic viscosity of $34,95 \cdot 10^{-6}$ $m^2/s$ (34.95 cSt) (40°C) and $4,65 \cdot 10^{-6}$ $m^2/s$ (4.650 cSt) (100°C), a specific gravity of 0.9409 (15/4°C) and a refractive index of 1.5042 ($n_D^{20}$). The product was measured for traction coefficient in the range of 40 to 140°C. The results are shown in Fig. 1.

REFERENCE EXAMPLE 6

The procedure of Reference Example 5 was repeated with the exception that 15 g of a nickel/diatomaceous earth catalyst (N-113 produced by Nikki Kagaku Co., Ltd.) was used in place of 12 g of the 5% ruthenium-carbon catalyst, and the reaction temperature was changed from 150°C to 250°C, to thereby obtain 155 g of 1-cyclohexyl-1-decalylethane (a mixture of 65% of 1-cyclohexyl-1-(1-decalyl)ethane and 35% of 1-cyclohexyl-1-(2-decalyl)ethane) having a kinematic viscosity of $25{,}33 \cdot 10^{-6}$ m²/s (25.33 cSt) (40°C) and $3{,}749 \cdot 10^{-6}$ m²/s (3.749 cSt) (100°C), a specific gravity of 0.9326 (15/4°C) and a refractive index of 1.5009 ($n_D^{20}$). The product was measured for traction coefficient in the range of 40 to 140°C. The results are shown in Fig. 2.

COMPARATIVE EXAMPLE 6

The procedure of Comparative Example 5 was repeated with the exception that 15 g of a nickel/diatomaceous earth catalyst (N-113 produced by Nikki Kagaku Co., Ltd.) was used in place of 12 g of the 5% ruthenium-carbon catalyst, and the reaction temperature was changed from 150°C to 250°C, to thereby obtain 155 g of 1-cyclohexyl-1-decalylethane (a mixture of 95% of 1-cyclohexyl-1-(2-decalyl)ethane and 5% of 1-cyclohexyl-1-(1-decalyl)ethane) having a dynamic viscosity of $21{,}89 \cdot 10^{-6}$ m²/s (21.89 cSt) (40°C) and $3{,}681 \cdot 10^{-6}$ m²/s (3.681 cSt) (100°C), a specific gravity of 0.9301 and a refractive index of 1.4998 ($n_D^{20}$). The product was measured for traction coefficient in the range of 40 to 140°C. The results are shown in Fig. 2.

## Claims

1. A fluid composition comprising from 30 to 65% by weight of 1-phenyl-1-(α-naphthyl)ethane and from 70 to 35% by weight of 1-phenyl-1-(β-naphthyl)ethane provided provided that the sum of the amounts of 1-phenyl-1-(α-naphthyl) ethane and 1-phenyl-1-(β-naphthyl) ethane are 100%.

2. A process for preparing the fluid composition of claim 1 which comprises feeding naphthalene and styrene in a molar ratio of 1:1 to 10:1 and then reacting them in the presence of a clay at a temperature of from 50 to 180°C.

3. The process as claimed in Claim 2 wherein the clay is a clay selected from the group consisting of activated clay, bentonite, montmorillonite, acid clay and kaolin.

4. The use of the fluid composition of claim 1 as an electric insulating oil on as a heat medium.

## Patentansprüche

1. Flüssiges Gemisch, das umfaßt 30 bis 65 Gew.-% an 1-Phenyl-1-(α-naphthyl)-ethan und 70 bis 35 Gew.-% 1-Phenyl-1-(β-naphthyl)ethan, mit der Maßgabe, daß die Summe der Mengen von 1-Phenyl-1-(α-naphthyl)ethan und 1-Phenyl-1-(β-naphthyl)ethan 100 % beträgt.

2. Verfahren zur Herstellung des flüssigen Gemisches nach Anspruch 1, das umfaßt das Zuführen von Naphtalin und Styrol in einem molaren Verhältnis von 1:1 zu 10:1 und im Anschluß deren Umsetzen in Gegenwart von Tonerde bei einer Temperatur von 50 bis 180° C.

3. Verfahren nach Anspruch 2, worin die Tonerde eine Tonerde ist, ausgewählt aus der Gruppe, die besteht aus aktivierter Tonerde, Bentonit, Montmorrilonit, saurer Tonerde und Kaolin.

4. Die Verwendung des flüssigen Gemisches nach Anspruch 1 als elektrisches Isolationsöl oder als ein Hitzemedium.

## Revendications

1. Composition fluide comprenant 30 à 65 % en poids de 1-phényl-1-(α-naphthyl)éthane et 70 à 35 % en poids de 1-phényl-1-(β-naphthyl)éthane, étant entendu que la somme des quantités de 1-phényl-1-(α-naphthyl)éthane et de 1-phényl-1-(β-naphthyl)éthane est de 100 %.

2. Procédé pour préparer la composition fluide de la revendication 1, qui consiste à fournir du naphthalène et du styrène en un rapport molaire de 1:1 à 10:1, puis à les faire réagir en présence d'une argile à une température de 50 à 180°C.

3. Procédé selon la revendication 2, dans lequel l'argile est une argile choisie dans le groupe formé par

une argile activée, la bentonite, la montmorillonite, une argile acide et le kaolin.

4. Utilisation de la composition fluide de la revendication 1 comme huile diélectrique ou comme milieu thermique.

FIG. 1

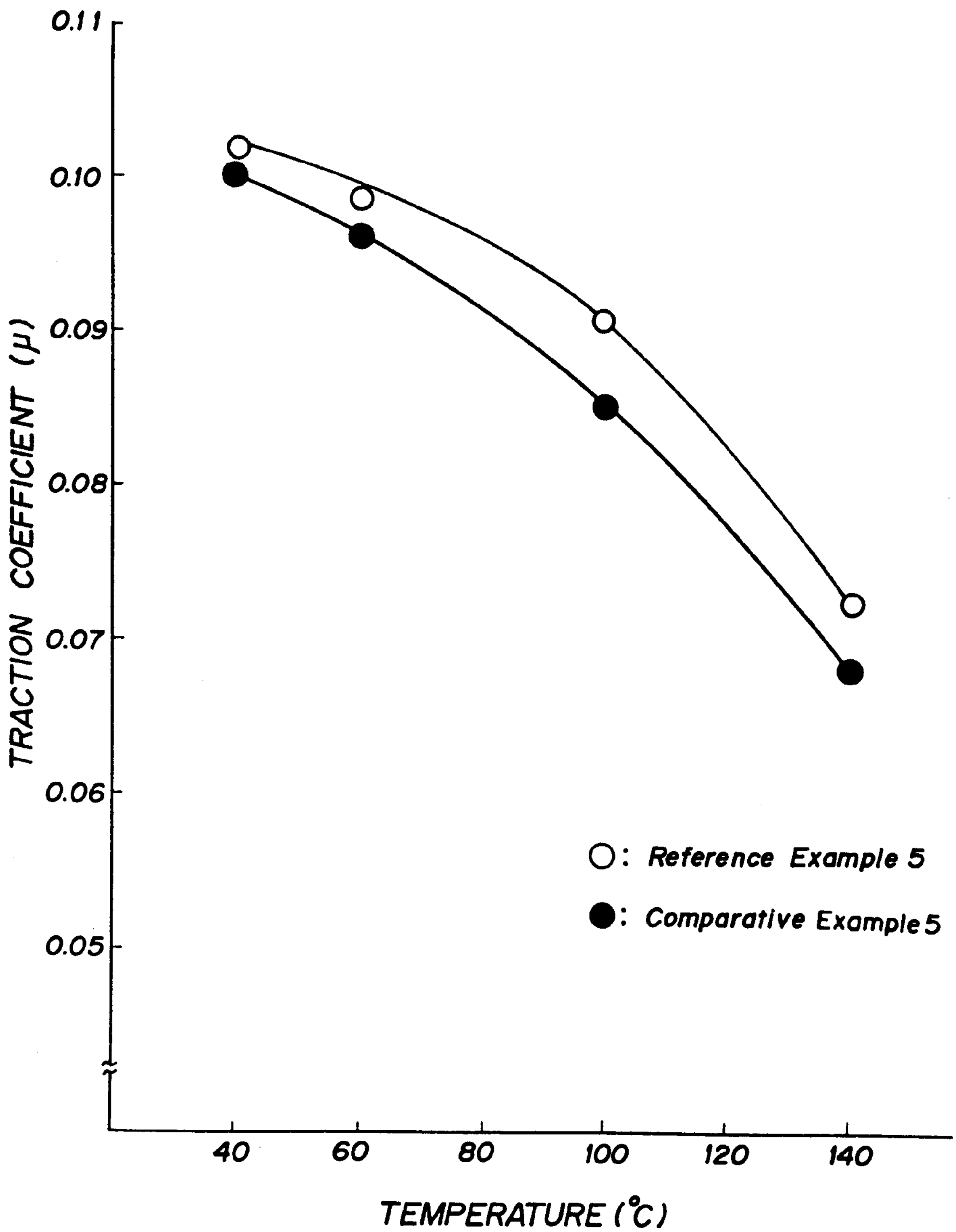
0.11
0.10
0.09
0.08
0.07
0.06
0.05
TRACTION COEFFICIENT (μ)
40
60
80
100
120
140
TEMPERATURE (°C)
○: Reference Example 5
●: Comparative Example 5

FIG. 2

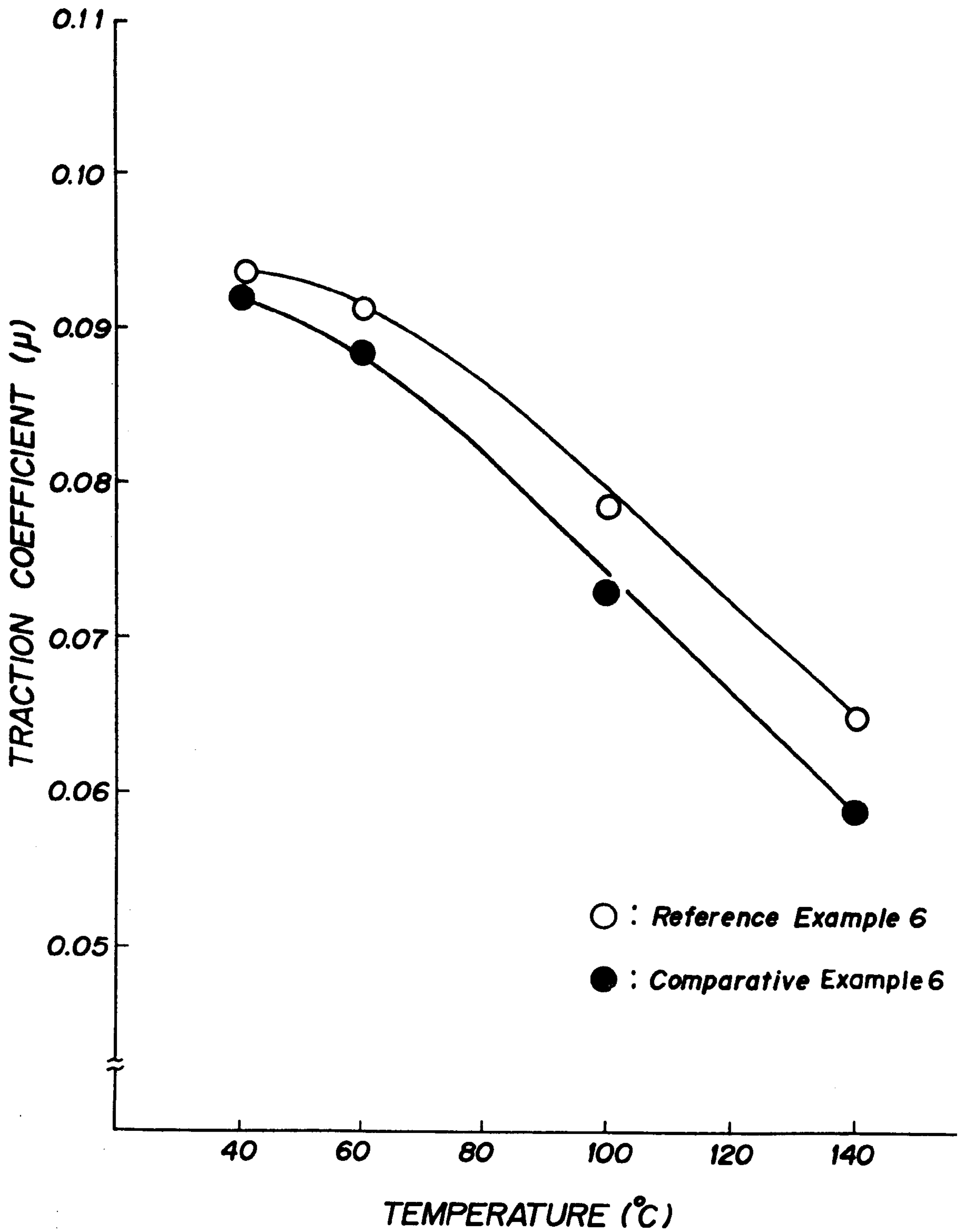
0.11
0.10
0.09
0.08
0.07
0.06
0.05
TRACTION COEFFICIENT (μ)
40
60
80
100
120
140
TEMPERATURE (°C)
○ : Reference Example 6
● : Comparative Example 6